# EUROPEAN PATENT APPLICATION

(11) **EP 4 643 863 A1**
(43) Date of publication of application: **05.11.2025**
(21) Application number: 22969798.2
(22) Date of filing: 30.12.2022
(51) Int. Cl.: A61K 31/5377, A61P 35/00

(54) **PHARMACEUTICAL COMPOSITION AND USE THEREOF**

(71) Applicant: Tsinghua University, Beijing 100084 (CN)
(72) Inventor: ZHANG, Wei, Beijing 100084 (CN)
(74) Representative: Ullrich & Naumann PartG mbB
(86) International application number: PCT/CN2022/144029
(87) International publication number: WO 2024/138655

(57) **Abstract**

A pharmaceutical composition, comprising panobinostat and tazemetostat. Combination of tazemetostat which is an EZH2 inhibitor with panobinostat, pembrolizumab and ipilimumab is clinically used for treating patients suffering from later-stage diffuse midline glioma, H3K27-altered, accompanied with leptomeningeal/pia mater spinalis spread, and patients suffering from primary, relapsing and progressing diffuse midline glioma, H3K27-altered. The pharmaceutical composition has remarkable curative effects, can obviously inhibit tumor growth, reduce the tumor volume, improve the life quality for patients, and prolong the survival time of patients.

## Description

### FIELD

The present disclosure relates to the field of pharmaceutical technology, and in particular to a pharmaceutical composition and use thereof.

### BACKGROUND

According to the World Health Organization Classification of Tumors of the Central Nervous System, 2021, 5th edition, Diffuse midline glioma, H3K27-altered is a pediatric-type diffuse high-grade glioma usually arising in the midline region of the central nervous system, which mostly affects thalamus, pons, spinal cord, etc. Surgical resection is challenging and biopsy is often performed in most cases. To date, no conventional, targeted or immune therapy has been shown to improve the overall survival of the patient. Internationally, treatment guideline is absent. Therefore, effective treatment strategies need to be explored urgently. The prognosis for patients with diffuse midline gliomas, H3K27-altered, is extremely poor, with a 2-year survival rate of less than 10%. Of particular note, diffuse midline glioma, H3K27-altered, arising from spinal cord with leptomeningeal/ spinal meningeal metastasis is rarely reported. In the historical literature, the patients with high-grade glioma with leptomeningeal/ spinal meningeal metastasis were often excluded from clinical trials due to worse prognosis, with a median survival of only 1.6-3.8 months.

### SUMMARY

In view of this, the technical problem to be solved by the present disclosure is to provide a pharmaceutical composition and use thereof, wherein the pharmaceutical composition provided in the present disclosure can be used for treating diffuse midline glioma, H3K27 altered, which can inhibit tumor growth, reduce tumor volume, significantly improve the life quality of the patient, and prolong the survival period of the patient.

The present disclosure provides a pharmaceutical composition comprising a HDAC inhibitor and an EZH2 inhibitor.

The inventor of the present disclosure inventively found that the combination of a histone deacetylase inhibitor (HDAC) and an EZH2 inhibitor for the treatment of tumor, particularly diffuse midline gliomas, H3K27 altered, shows a significant clinical efficacy. In some specific embodiments, the mass ratio of the HDAC inhibitor to EZH2 inhibitor is 100-200:1000-3000, preferably 100-200:1200-2800, more preferably 100-200:1500-2500.

In some specific embodiments, the HDAC inhibitor includes, but no limited to, one or more of panobinostat, vorinostat, romidepsin, belinostat, and chidamide, preferably panobinostat, which was approved by the U.S. Food and Drug Administration in 2015 for the treatment of multiple myeloma. Studies have shown that panobinostat can penetrate the blood-brain barrier and is a promising drug for treatment of diffuse midline glioma, H3K27 altered, but it did not significantly improve 6-month progression-free survival period for patients with recurrent glioblastoma and anaplastic glioma.

In some specific embodiments, the EZH2 inhibitor includes, but no limited to, tazemetostat, a small molecule EZH2 inhibitor, which was approved by the U.S. Food and Drug Administration in 2020 for the treatment of epithelioid sarcoma, and has not been subjected to clinical trials in glioma patients so far. The inventors of the present disclosure have inventively found that the EZH2 inhibitor can be used as an epigenetic drug for the treatment of patients with diffuse midline glioma, H3K27 altered, in clinical. In particular, the combination of tazemetostat and panobinostat has a significant efficacy on the treatment of advanced diffuse midline glioma, H3K27 altered, with leptomeningeal/ spinal meningeal metastasis, and on the treatment of primary, recurrent, or progressed diffuse midline glioms, H3K27 altered, which can significantly inhibit tumor growth, reduce tumor volume, improve life quality of patients and prolong their survival.

In some specific embodiments, the pharmaceutical composition comprises panobinostat and tazemetostat. The mass ratio of the panobinostat to tazemetostat is 100-200:1000-3000, preferably 100-200:1200-2800, more preferably 100-200:1500-2500.

In some specific embodiments, the pharmaceutical composition further comprises an inhibitor selected from the group consisting of a PD-1 inhibitor, a PD-L1 inhibitor, a CTLA-4 inhibitor, a VEGF inhibitor, and a combination thereof.

In some specific embodiments, the PD-1 inhibitor is selected from the group consisting of pembrolizumab, nivolumab, tislelizumab, toripalimab, sintilimab, camrelizumab, zimberelimab, penpulimab, serplulimab, pucotenlimab, and a combination thereof, preferably pembrolizumab.

In some specific embodiments, the PD-L1 inhibitor is selected from the group consisting of atezolizumab, durvalumab, envafolimab, sugemalimab, avelumab, and a combination thereof.

In some specific embodiments, the CTLA-4 inhibitor is selected from the group consisting of ipilimumab, tremelimumab, and a combination thereof, preferably ipilimumab.

In some specific embodiments, the VEGF inhibitor is selected from the group consisting of bevacizumab, ramucirumab, lucentis, aflibercept, and a combination thereof, preferably bevacizumab.

In some specific embodiments, the pharmaceutical composition further comprises pembrolizumab, which is a PD-1 inhibitor as an immune checkpoint inhibitor. The mass ratio of the panobinostat to the pembrolizumab is 100-200:50-400, preferably 100-200:100-350, more preferably 100-200:150-300.

In some specific embodiments, the pharmaceutical composition further comprises ipilimumab, which is a CTLA-4 inhibitor as an immune checkpoint inhibitor. Found by the present disclosure is that the pembrolizumab and ipilimumab in combination with panobinostat and tazemetostat has a significant efficacy for treating diffuse midline glioma, H3K27 altered,, which can inhibit tumor growth, reduce tumor volume, significantly improve the life quality the patient, and prolong the survival of the patient. The mass ratio of the panobinostat to the ipilimumab is 100-200:10-100, preferably 100-200:20-80.

In some specific embodiments, the pharmaceutical composition further comprises bevacizumab, which is a recombinant humanized monoclonal antibody targeting VEGF. In some specific embodiments, the mass ratio of the panobinostat to the bevacizumab is 100-200:300-1000, preferably 100-200:350-800, more preferably 100-200:400-700.

In some specific embodiments, the pharmaceutical composition comprises panobinostat, tazemetostat, and pembrolizumab. In some specific embodiments, the pharmaceutical composition comprises panobinostat, tazemetostat, and ipilimumab. In some specific embodiments, the pharmaceutical composition comprises panobinostat, tazemetostat, pembrolizumab, and ipilimumab. In some specific embodiments, the pharmaceutical composition comprises panobinostat, tazemetostat, pembrolizumab, ipilimumab, and bevacizumab.

In some specific embodiments, the pharmaceutical composition comprises an EZH2 inhibitor and a PD-1 inhibitor or a PD-L1 inhibitor, wherein the mass ratio of the EZH2 inhibitor to PD-1 inhibitor or PD-L1 inhibitor is 1000-3000:50-400. In some specific embodiments, the pharmaceutical composition comprises an EZH2 inhibitor and a CTLA-4 inhibitor, wherein the mass ratio of EZH2 inhibitor to CTLA-4 inhibitor being 1000-3000:10-100. In some specific embodiments, the pharmaceutical composition comprises an EZH2 inhibitor, a PD-1 inhibitor or PD-L1 inhibitor, and a CTLA-4 inhibitor, wherein the mass ratio of the EZH2 inhibitor, the PD-1 inhibitor or PD-L1 inhibitor, and the CTLA-4 inhibitor is 1000-3000:50-400:10-100. In some possible embodiments, the pharmaceutical composition comprises an inhibitor selected from the group of an EZH2 inhibitor, a PD-1 inhibitor or PD-L1 inhibitor, a CTLA-4 inhibitor, a VEGF inhibitor, and a combination thereof. Wherein the mass ratio of the EZH2 inhibitor, the PD-1 inhibitor or PD-L1 inhibitor, the CTLA-4 inhibitor and the VEGF inhibitor is 1000-3000:50-400:10-100:300-1000. In some specific embodiments, the pharmaceutical composition comprises tazemetostat and pembrolizumab. In some specific embodiments, the pharmaceutical composition comprises tazemetostat and ipilimumab. In some specific embodiments, the pharmaceutical composition comprises tazemetostat, pembrolizumab and ipilimumab. In some specific embodiments, the pharmaceutical composition comprises tazemetostat, pembrolizumab, ipilimumab, and bevacizumab.

In some specific embodiments, the pharmaceutical composition comprises a HDAC inhibitor, and a PD-1 inhibitor or PD-L1 inhibitor, wherein the mass ratio of the HDAC inhibitor to the PD-1 inhibitor or PD-L1 inhibitor is 100-200:50-400. In some specific embodiments, the pharmaceutical composition comprises a HDAC inhibitor and a CTLA-4 inhibitor, wherein the mass ratio of the HDAC inhibitor and the CTLA-4 inhibitor is 100-200:10-100. In some specific embodiments, the pharmaceutical composition comprises a HDAC inhibitor, a PD-1 inhibitor or PD-L1 inhibitor, and a CTLA-4 inhibitor, wherein the mass ratio of the HDAC inhibitor, the PD-1 inhibitor or PD-L1 inhibitor, and the CTLA-4 inhibitor is 100-200:50-400:10-100. In some specific possible embodiments, the pharmaceutical composition comprises one selected from the group consisting of a HDAC inhibitor, a PD-1 inhibitor or PD-L1 inhibitor, a CTLA-4 inhibitor and a VEGF inhibitor, or a combination thereof; wherein the mass ratio of the HDAC inhibitor, the PD-1 inhibitor or PD-L1 inhibitor, the CTLA-4 inhibitor and the VEGF inhibitor is 100-200:50-400:10-100:300-1000. In some specific embodiments, the pharmaceutical composition comprises panobinostat and pembrolizumab. In some specific embodiments, the pharmaceutical composition comprises panobinostat and ipilimumab. In some specific embodiments, the pharmaceutical composition comprises panobinostat, pembrolizumab and ipilimumab. In some specific embodiments, the pharmaceutical composition comprises panobinostat, pembrolizumab, ipilimumab, and bevacizumab.

The present disclosure further provides use of the pharmaceutical composition in the manufacture of a medicament against a tumor. In some specific embodiments, the tumor is a central nervous system tumor. In some specific embodiments, the tumor is a glioma. In some specific embodiments, the tumor is a pediatric-type diffuse high-grade glioma. In some specific embodiments, the tumor is a diffuse midline glioma, H3K27 altered. These tumor names are derived from the 2021 WHO Classification of Tumors of the Central Nervous System (fifth edition).

H3K27M as the most common H3K27 alteration, refers to a substitution of lysine by methionine at position 27 in histone 3, which is a classic "oncogenic histone". It can drive epigenomic reprogramming in diffuse midline glioma, H3K27 altered, which exhibits a distinct chromatin landscape characterized by extensive loss of H3K27 trimethylation and up-regulation of H3K27 acetylation. H3K27 acetylation is often located in the intergenic region, and promotes the expression of endogenous retroviral elements, and epigenetic drugs can further up-regulate H3K27 acetylation. This viral mimicry can prime innate immune responses and promote immune cell activation. Based on this, in the present disclosure, immunotherapy based on epigenetic drugs is applied to treat diffuse midline glioma, H3K27 altered, in particular based on the combination of epigenetic drugs and immune checkpoint inhibitors, which can provide a survival benefit to patients with glioma.

The experimental results show that after the treatment regimen comprising resection of recurrent thoracic lesions with maximal safety in combination with panobinostat, tazemetostat, pembrolizumab, ipilimumab, and bevacizumab was used for treating diffuse midline glioma, H3K27 altered, all tumor lesions volume in the patients showed a sustained shrinkage up to the data cut-off (on day 371), with those in the suprasaddle area (T1) and dorsal medulla (T2) measured to be 137 and 14 mm³, corresponding to >94% reduction compared with baseline volume. Enhancement signals in the leptomeningeal/ spinal meninges and periventricular were no longer detectable on enhanced MRI imaging on day 367. The patient had significantly improved life quality through the treatment and went back to normal life activities at the cycle 11 of treatment.

Samples of cerebrospinal fluid (CSF) from the patient were collected on days 26, 72, 169, 321, and 340, and subjected to proteome analysis by mass spectrometry. A total of 1812 proteins were identified, of which 1118 were differentially expressed across the time points and clustered into 20 groups based on expression patterns. Four clusters of consistently changed proteins were focused on. The results indicated that the expression of proteins associated with tumor angiogenesis, metastasis and proliferation, such as VEGFA, collagen family, and laminin family, was remarkably decreased since day 26 of the treatment. Proteins related to tumor invasion, migration, epithelial-mesenchymal transition and stemness, such as MMP9, VIM and NES, showed a steady decline since day 72 of the treatment. In addition, the expression of proteins including MRC1, a marker of M2-like tumor-associated macrophages; VISG4, a negative regulator of cytotoxic T lymphocytes activation; and PLAUR, a marker of immunosuppression in glioma, was decreased significantly since day 26 of the treatment. ICAM1 and ICAM2, proteins associated with immune cell migration and activation, were increased since day 72 of the treatment. The expression of proteins related to immune activation and enhanced immune response such as SELL, LYZ and ATRN was significantly increased since day 169 of the treatment, and maintained until the last sampling day.

This patient's tumor progressed malignantly at two new locations after data cut-off. The overall survival time of the patient was 20 months, and the survival time of the patient after epigenetic drug-based immunotherapy was 16 months. This therapy was further applied in 3 additional patients with diffuse midline glioma, H3K27 altered, all of which showed a significant clinical efficacy.

### BRIEF DESCRIPTION OF DRAWINGS

FIG. 1 shows the results of pathological diagnosis of patient 1.
FIG. 2 shows the treatment regimen and the response of the tumor to the treatment regimen.
FIG. 3 shows the response of patient-derived tumor cells to epigenetic therapy.
FIG. 4 shows the molecular response of patient-derived tumor cells to epigenetic therapy.
FIG. 5 shows intracranial LM imaging changes (sagittal view).
FIG. 6 shows intracranial LM imaging changes (axial view).
FIG. 7 shows intracranial LM imaging changes (coronal view).
FIG. 8 shows thoracic tumor imaging changes (sagittal view).
FIG. 9 shows the results of neurocognitive function score.
FIG. 10 shows the clustering trends of differential protein expression in cerebrospinal fluid.
FIG. 11 shows the full list of differential protein expression in cerebrospinal fluid.
FIG. 12 shows the clinical efficacy of patient 2.
FIG. 13 shows the clinical efficacy of patient 3.
FIG. 14 shows the clinical efficacy of patient 4.
FIG. 15 shows the clinical efficacy of patient 5.

### DETAILED DESCRIPTION

The present disclosure provides a pharmaceutical composition and use thereof, which can be achieved by those skilled in the art through appropriately improving the process parameters in light of the present disclosure. It should be particularly indicated that, all similar replacements and changes are obvious for those skilled in the art, and are deemed to be included in the present disclosure. The method and use of the present disclosure have been described through preferred embodiments, and those skilled apparently can make modifications or appropriate changes and combinations of the method and use herein without departing from the content, spirit and scope of the present disclosure to realize and apply the technology of the present disclosure.

### Examples

### Example 1

### Materials and method

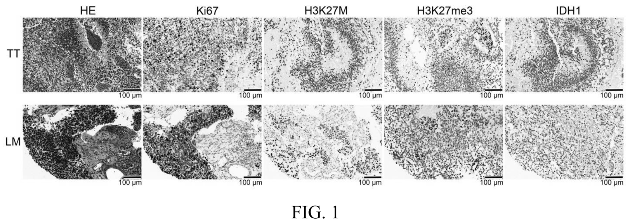

Patient 1 was a 28-year-old male diagnosed with primary diffuse midline glioma, H3K27 altered, in the thoracic cord on September 13th 2020 (see Table 1 for detailed information). After tumor resection without any adjuvant therapy, the patient recovered well with minor neurological deficits. Three months after the surgery, the patient developed leg weakness, hiccups, and lethargy, and these symptoms progressively worsened. On January 11th, 2021 (on day 1), due to confusion and sudden coma, the patient was admitted to Beijing Tsinghua Changgung Hospital with Glasgow Coma Scale score of 8/15 on admission. Magnetic resonance imaging (MRI) showed thoracic tumor recurrence and leptomeningeal/spinal meningeal metastasis involving brain and spinal cord. Subtotal resection of his recurrent thoracic lesion and biopsy of his lesion in dorsal medulla were performed (on day 5). Pathologic diagnosis for lesions in both locations was diffuse midline glioma, H3K27 altered (WHO Classification, Grade 4) (see FIG. 1). FIG.1 shows the results of pathological diagnosis of patient 1. FIG.1 shows representative staining results of the recurrent thoracic tumor (TT, upper side) and leptomeningeal metastasis in dorsal medulla (LM, lower side) in pathological diagnosis. Neovascularization as well as foci of hemorrhage and necrosis are observed around tumor cells by HE staining. Both lesions had a high Ki-67 score, and staining shows that tumor cells are positive for H3K27M and negative for H3K27me3 and IDH1. In FIG. 1, TT represents thoracic tumor, LM represents leptomeningeal metastasis, HE represents hematoxylin and eosin, H3K27me3 represents trimethylated H3K27, and IDH1 represents isocitrate dehydrogenase 1.

**Table 1 Patient Details**

| Numb er for patient | Gender | Age | Primary/Recur rent | Tumor location | Histo ne H3 | OS (month) |
|---|---|---|---|---|---|---|
| 1 | Male | 28 | Recurrent | Thoracic Cord with leptomeningeal/ spinal meningeal metastasis | H3F3 A mut | 20 |
| 2 | Male | 53 | Recurrent | Cervical Cord | H3F3 A mut | 16+ |
| 3 | Fema le | 25 | Recurrent | Left Thalamus | H3F3 A mut | 27 |
| 4 | Male | 44 | Recurrent | Right Thalamus | H3F3 A mut | 16 |
| 5 | Male | 25 | Recurrent | Cervical Cord with Multifocal Brain Metastases | H3F3 A mut | 43 |

### Treatment regimen

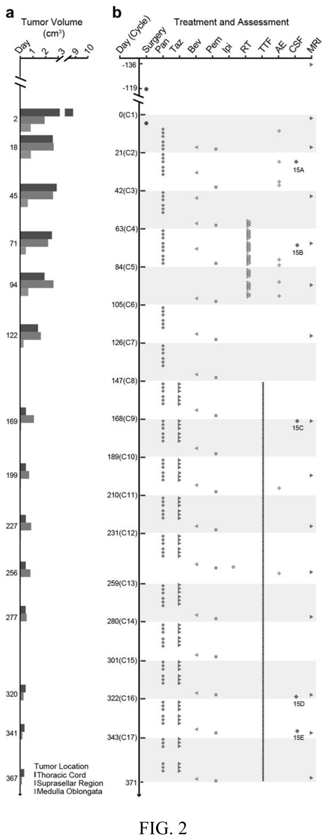

A treatment regimen for this patient was approved by the Ethics Committee of Beijing Tsinghua Changgung Hospital for a compassionate purpose. Written informed consent for participation was given by the patient and his family. No commercial sponsor was involved throughout the treatment. The treatment regimen is shown in FIG. 2, which shows the treatment regimen and the response of the tumor to the treatment regimen. Wherein, FIG. 2(a) shows the changes in tumor volume at three locations of thoracic cord, suprasaddle area, and medulla oblongata, on each MRI assessment that were measured by ITK-SNAP¹⁵; and FIG. 2(b) shows the timeline for treatment, including epigenetic drugs (panobinostat and tazemetostat), VEGFA inhibitor bevacizumab, immune checkpoint blockades (pembrolizumab and ipilimumab), radiotherapy, and tumor treating fields. First occurrences of treatment-related adverse events (see Table 2 for details), analysis of cerebrospinal fluid, and MRI assessment are also dated on the timeline. Timeline spanned from January 11, 2021 (On day 1), 4 months after the initial diagnosis, to data cutoff on January 16, 2022 (On day 371), comprising 17 treatment cycles of 21 or 28 days. Cerebrospinal fluid samples were denoted 15A-E in a chronological order for ease of reference (FIG. 2b). Wherein, Pan represents panobinostat; Taz represents tazemetostat; Bev represents bevacizumab; Pem represents pembrolizumab; Ipi represents ipilimumab; RT represents radiotherapy; TTF represents tumor treating fields; AE represents adverse events; CSF represents cerebrospinal fluid; MRI represents magnetic resonance imaging; LM represents leptomeningeal metastasis; VEGFA represents vascular endothelial growth factor A; and ICB represents immune checkpoint blockade.

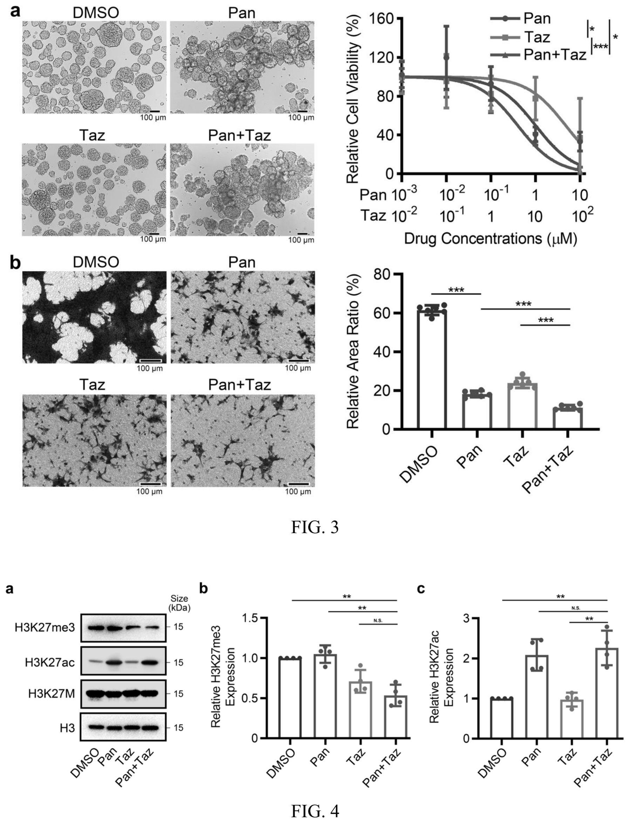

Specifically, 3 weeks is one treatment cycle. Panobinostat was administered orally 20 mg 3 times per week (on days 1, 3, 5, 8, 10, and 12) in week 1 and 2, 10 mg/kg of bevacizumab and 200 mg of pembrolizumab were injected intravenously in week 3. Because of the critical condition of the patient on admission, bevacizumab was administered once every two weeks through treatment cycles 1-4. The patient received radiotherapy of whole brain (41.4 Gy in 23 fractions) and local enhancement radiotherapy in saddle area (10 Gy in 5 fractions) during treatment cycles 3-5. Due to grade 3 adverse events of leukopenia, neutropenia, and weight loss, panobinostat was suspended in cycle 5 (FIG. 2(b) and Table 2). Vitro cell viability assay and cell invasion assay were performed, indicating that THG15 cells (derived from the thoracic tumor of patient 1) were more sensitive to panobinostat in combination with tazemetostat treatment, compared to either agent alone (see FIG. 3 and FIG. 4). Beginning with cycle 8, treatment with oral 400 mg tazemetostat and tumor treating fields were initiated concurrently with panobinostat. In cycle 11, the oral dose of panobinostat was reduced to 10 mg due to satisfactory therapeutic response as well as increased alkaline phosphatase in peripheral blood (FIG. 2(a), on day 199, Table 2 and Table 5), but was increased back to 20 mg since cycle 12 due to later LM progression (FIG. 2, On day 227, Table 5). Intravenous injection of ipilimumab (50 mg) was introduced into the treatment regimen in cycle 12 for the following considerations: 1. EZH2 inhibitor could improve the efficacy of an inhibitor of anti-cytotoxic T lymphocyte antigen 4 (CTLA-4) and overcome resistance to immune checkpoint blockade. 2. Periventricular LM had slightly progressed in cycle 11. 3. The volume of all lesions was sufficiently reduced after oral drug adjustment (FIG. 2 and Table 5). 4. Combination of programmed cell death protein 1 (PD-1) and CTLA-4 inhibitors have been reported to increase the incidence of immune-related adverse events which may result in therapy suspension or discontinuation. Due to fatigue (CTCAE grade 2) occurred to the patient, cycle 13 was delayed by one week during which no pharmacological interventions were performed, and the dose of tazemetostat was reduced to 200 mg since cycle 13. The three-week treatment cycle was extended to four week since cycle 17. Data collection was cut off on day 371 (12 April, 2022).

### Safety and adverse event

Ten adverse events of grades 1-3 that may be related to treatment are shown in Table 2. Grade 3 adverse events included anemia, leukopenia, neutropenia, and weight loss, all of which were alleviated after blood transfusion or panobinostat suspension. Adverse events of low grades included diarrhea, fatigue, thrombocytopenia, elevated transaminases and alkaline phosphatase. There were no adverse events of grade 4 or discontinuation of treatment due to adverse events during the full course of treatment.

**Table 2 Treatment-related adverse events**

| Day (Cycle) | Event | Class of System or Organ | Baseline Grade | Grade of adverse event | Treatment Strategy | Outcome |
|---|---|---|---|---|---|---|
| 9 (C1) | Diarrhea | Gastrointestinal disorders | Normal | 1 | Drug therapy | Resolved |
| 26 (C2) | Elevated level of alanine aminotransferase | Hepatic dysfunction | Normal | 2 | Conservative treatment | Resolved |
| 37 (C2) | Anemia | Blood and lymphatic system disorders | Normal | 3 | Transfusion | Resolved |
| 39 (C2) | Elevated level of aspartate aminotransferase | Hepatic dysfunction | Normal | 1 | Conservative treatment | Resolved |
| 80 (C4) | Decreased platelet count | Blood and lymphatic system disorders | Normal | 2 | Transfusion | Improved |
| 83 (C4) | Decreased white blood cell count | Blood and lymphatic system disorders | Normal | 3 | Panobinostat suspension | Resolved |
| 92 (C5) | Decreased neutrophil count | Blood and lymphatic system disorders | Normal | 3 | Panobinostat suspension | Resolved |
| 100 (C5) | Weight loss | Metabolism and nutrition disorders | 1 | 3 | Panobinostat suspension | Resolved |
| 206 (C10) | Elevated level of alkaline phosphatase | Routine laboratory test | Normal | 2 | Conservative treatment | Resolved |
| 253 (C12) | Fatigue | General disorders and administration site conditions | 1 | 2 | Conservative treatment | Resolved |

### Pathological diagnosis

Pathological tissues were fixed with formalin, embedded in paraffin, and sectioned. Tissue sections were examined by HE (hematoxylin and eosin) and immunohistochemistry staining as described in Yao, J., Wang, L., Ge, H., Yin, H. & Piao, Y. Diffuse midline glioma with H3 K27M mutation of the spinal cord: A series of 33 cases. Neuropathology 41, 183-190, doi:10.1111/neup.12714 (2021). For sources of anti-Ki-67 antibody, anti-H3K27M antibody, anti-H3K27me3 antibody, anti-isocitrate dehydrogenase 1 (IDH1) R132H mutant antibody and secondary antibodies, see Yao, J., Wang, L., Ge, H., Yin, H. & Piao, Y. Diffuse midline glioma with H3 K27M mutation of the spinal cord: a series of 33 cases. Neuropathology 41, 183-190, doi:10.1111/neup.12714 (2021). Immunoreaction was detected with a Leica Bond automated staining processor.

### Culture of patient-derived tumor cell

Patient-derived tumor cell were obtained with the approval of the Ethics Committee of the Beijing Tsinghua Changgung Hospital in compliance with informed consent of the patient and his family. Surgical sample derived from the thoracic tumor of patient 1 was named as THG15 (Tsinghua Glioma 15). The specific procedure was referred to Zhang, W. *et al.* Combination of oncolytic herpes simplex viruses armed with angiostatin and IL-12 enhances antitumor efficacy in human glioblastoma models. Neoplasia 15, 591-599, doi:10.1593/neo.13158 (2013), and Chen, W. J. et al. The different role of YKL-40 in glioblastoma is a function of MGMT promoter methylation status. Cell Death Dis 11, 668, doi: 10.1038/s41419-020-02909-9 (2020). Briefly, the tissues were mechanically minced and then digested with 0.1% trypsin (Hyclone) and 10 U/ml DNasel (Roche) at 37°C for 45 min. The tissues were passed through a 100-µm cell strainer after lysis of red blood cells and several washes. Cells that that passed through the filter were collected and added with EF medium composed of Neurobasal medium, 3 mM L-glutamine, 1×B27 supplement, 0.5×N2 supplement, 2 µg/mL heparin, 20 ng/mL recombinant human EGF (Sigma-Aldrich), 20 ng/mL recombinant human FGF2 (Sigma-Aldrich), and 0.5×antibiotic antimycotic complex. Fresh EF medium was replenished every 3 days during cell culture. Tumor cell spheres were dissociated using NeuroCult Chemical Dissociation kit (StemCell), and cells were passaged.

### Cell viability assay

Panobinostat (LBH589) and Tazemetostat (EPZ-6438) were purchased from Selleck Chemicals and dissolved in 0.1% DMSO before use. About 5,000 THG15 cells were seeded into each well of a 96-well plate and cultured in EF medium for 24 hours prior to drug intervention. Cells were then incubated with different concentrations of panobinostat, tazemetostat, or both in triplicates for 72 hours. According to the instructions, cell viability was determined by using a CCK-8 kit (GK10001), and the optical density was measured at 450 nm using a SPARK reader (TECAN). To determine the inhibitory effect of each intervention, viability of drug-intervened cells was compared with that of untreated cells. A standard dose-response curves with Hill slope of -1.0 were generated and corresponding half maximal inhibitory concentrations (IC50) was calculated on Prism 9 (GraphPad) based on the results of two independently assays.

### Transwell migration assay

About 5x10⁵ THG15 cells were resuspended in 100 µL DMEM medium, and seeded into the upper chamber of each transwell insert in a 24-well plate. DMEM+20% FBS (Gibco) was added to the lower chamber. 1 µM panobinostat, 50 µM tazemetostat, or both were set up as intervention group, with triplicates for each group. After 48 hours of incubation, the cells were fixed in 4% paraformaldehyde (Solarbio) and stained with 1% crystal violet (Solarbio). The upper surface of the upper chamber of the transwell was gently wiped with a cotton swab to remove cells that failed to migrate across the membrane. Residual cells were observed under an inverted fluorescence microscope (Ts2R-FL, Nikon). The relative area of the stained cells was quantified on Image J as average from three random views.

### Western blotting assay

To analyze epigenetic alterations of tumor cells after drug intervention, 5×10⁵ THG15 cells/well were seeded to a 6-well plate and incubated with 1 µM panobinostat, 50 µM tazemetostat, or both in triplicates for 48 hours. Cells intervened by drugs was observed in the bright field using an inverted fluorescence microscope (Ts2R-FL, Nikon), representative images were captured on NIS-Elements (Nikon) with auto-exposure and auto-white balance. Cells were lysed in RIPA buffer (Solarbio), and samples were prepared with 5×SDS-PAGE Sample Loading Buffer (Beyotime) diluted with PBS (Solarbio) at 4:1. Total protein levels of samples were semi-quantified on the SurePAGE^{™} gel (Genscript, Nanjing, China) using eStain^{™} L1 Protein Staining system (Genscript). After adjusting the loading volume according to the total protein level, electrophoresis was performed on a 20% Bis-Tris gel (Genscript). Separated proteins were transferred to an Immobilon^{®}-P PVDF membrane (Millipore) by using an eBlot^{®} L1 Fast Wet Transfer System (Genscript). The membrane was blocked with 5% skimmed milk prepared with TBST consisting of 1×TBS (Lablead) and 0.1% TWEEN 20 (VWR), then incubated with anti-H3K27Me3 antibody, anti-H3K27Ac antibody, anti-H3K27M antibody, or anti-H3 antibody. After incubation with the corresponding secondary antibodies, blots were detected using SuperSignal^{™} West Pico PLUS Chemiluminescent System (Thermo Fisher Scientific) and imaged on ImageQuant LAS 4000 (GE). The relative intensities of blots were quantified with densitometric analysis on Image J.

### Magnetic resonance imaging and measurement of tumor volume

Magnetic resonance imaging of the brain and spinal cord was performed on 3.0 Tesla magnetic resonance imaging scanners, with T1-weighted, T2-weighted, and post-gadolinium T1-weighted images. Tumors were manually outlined by two neuroradiologists independently on the T1-weighted MRI images post-enhancement, and the tumor volumes were calculated with ITK-SNAP, and the calculation can be referred to Yushkevich, P. A. et al. User-guided 3D active contour segmentation of anatomical structures: significantly improved efficiency and reliability. Neuroimage 31, 1116-1128, doi:10.1016/j.neuroimage.2006.01.015 (2006).

### Assessment of health related quality of life

Quality of life of the patient was assessed according to two quality of life questionnaires (QLQ) developed by the European Organization for Research and Treatment of Cancer. The core QLQ-C30 consists of five functional scales, one global health and quality-of-life scale, three multi-item symptom scales, five single-item symptom measures, and a single subject measure of economic burden due to disease and treatment. The brain tumor specific QLQ-BN20 comprises four multi-item symptom scales and seven single-item symptom measures. Raw scale and measure scores were linearly transformed to a standardized score spanning 0 to 100 for ease of interpretation, wherein a higher score corresponded to a higher health functioning or level. All scales and indicators, except for physical functioning, were scored based on the average health state and quality of life in the week prior to data collection. The results are shown in Table 3 and Table 4, which shows the results of the QLQ-C30 evaluations, and the results of the QLQ-BN20 evaluations, respectively.

**Table 3 Results of QLQ-C30 evaluations**

| Score | Base line | C 1 | C 2 | C 3 | C 4 | C 5 | C 6 | C 7 | C 8 | C 9 | C 1 0 | C 1 1 | C 1 2 | C 1 3 | C 1 4 | C 1 5 | C 1 6 | C 1 7 |
|---|---|---|---|---|---|---|---|---|---|---|---|---|---|---|---|---|---|---|
| Physical function | 0 | 0 | 7 | 7 | 7 | 7 | 7 | 1 3 | 1 3 | 2 0 | 2 0 | 2 0 | 2 0 | 2 7 | 2 7 | 2 7 | 2 7 | 2 7 |
| Role function | 0 | 0 | 3 3 | 3 3 | 3 3 | 5 0 | 5 0 | 5 0 | 5 0 | 6 7 | 6 7 | 6 7 | 6 7 | 1 0 0 | 1 0 0 | 1 0 0 | 1 0 0 | 1 0 0 |
| Cognitive function | 33 | 3 3 | 6 7 | 8 3 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 |
| Emotional function | 0 | 3 3 | 4 2 | 6 7 | 7 5 | 7 5 | 7 5 | 1 0 0 | 1 0 0 | 1 0 0 | 1 0 0 | 1 0 0 | 1 0 0 | 1 0 0 | 1 0 0 | 1 0 0 | 1 0 0 | 1 0 0 |
| Social function | 0 | 3 3 | 3 3 | 6 7 | 8 3 | 8 3 | 8 3 | 8 3 | 8 3 | 1 0 0 | 1 0 0 | 1 0 0 | 1 0 0 | 1 0 0 | 1 0 0 | 1 0 0 | 1 0 0 | 1 0 0 |
| Global quality of life | 0 | 2 5 | 2 5 | 2 5 | 2 5 | 3 3 | 3 3 | 3 3 | 4 2 | 6 7 | 6 7 | 6 7 | 6 7 | 6 7 | 6 7 | 7 5 | 8 3 | 1 0 0 |
| Fatigue | 100 | 1 0 0 | 6 7 | 6 7 | 6 7 | 6 7 | 6 7 | 3 3 | 3 3 | 3 3 | 3 3 | 3 3 | 6 7 | 3 3 | 0 | 0 | 0 | 0 |
| Pain | 0 | 3 3 | 3 3 | 3 3 | 3 3 | 3 3 | 3 3 | 3 3 | 3 3 | 3 3 | 3 3 | 3 3 | 3 3 | 0 | 0 | 0 | 0 | 0 |
| Nausea and Vomiting | 33 | 1 7 | 1 7 | 6 7 | 1 0 0 | 6 7 | 6 7 | 6 7 | 6 7 | 3 3 | 3 3 | 3 3 | 0 | 0 | 0 | 0 | 0 | 0 |
| Dyspnea | 100 | 6 7 | 6 7 | 3 3 | 3 3 | 3 3 | 3 3 | 0 | 0 | 0 | 0 | 0 | 0 | 0 | 0 | 0 | 0 | 0 |
| Sleep disturbance | 100 | 6 7 | 3 3 | 0 | 0 | 0 | 0 | 0 | 0 | 0 | 0 | 0 | 0 | 0 | 0 | 0 | 0 | 0 |
| Appetite loss | 100 | 6 7 | 6 7 | 1 0 0 | 1 0 0 | 1 0 0 | 1 0 0 | 1 0 0 | 1 0 0 | 6 7 | 6 7 | 6 7 | 6 7 | 6 7 | 6 7 | 6 7 | 3 3 | 0 |
| Constipation | 0 | 0 | 0 | 0 | 0 | 0 | 0 | 0 | 0 | 0 | 0 | 0 | 0 | 0 | 0 | 0 | 0 | 0 |
| Diarrhea | 0 | 3 3 | 0 | 0 | 0 | 0 | 0 | 0 | 0 | 0 | 0 | 0 | 0 | 0 | 0 | 0 | 0 | 0 |
| Financial impact | 100 | 6 7 | 3 3 | 3 3 | 0 | 0 | 0 | 0 | 0 | 0 | 0 | 0 | 0 | 0 | 0 | 0 | 0 | 0 |

**Table 4 Results of QLQ-BN20 evaluations**

| Score | Base line | C 1 | C 2 | C 3 | C 4 | C 5 | C 6 | C 7 | C 8 | C 9 | C 1 0 | C 1 1 | C 1 2 | C 1 3 | C 1 4 | C 1 5 | C 1 6 | C 1 7 |
|---|---|---|---|---|---|---|---|---|---|---|---|---|---|---|---|---|---|---|
| Future uncertainty | 100 | 6 7 | 5 0 | 3 3 | 2 5 | 2 5 | 2 5 | 2 5 | 2 5 | 0 | 0 | 0 | 0 | 0 | 0 | 0 | 0 | 0 |
| Visual disorder | 33 | 3 3 | 3 3 | 2 2 | 2 2 | 2 2 | 0 | 0 | 0 | 0 | 0 | 0 | 0 | 0 | 0 | 0 | 0 | 0 |
| Motor dysfunction | 100 | 6 7 | 5 6 | 4 4 | 4 4 | 4 4 | 4 4 | 4 4 | 4 4 | 3 3 | 3 3 | 3 3 | 3 3 | 3 3 | 3 3 | 3 3 | 3 3 | 3 3 |
| Communicati on deficit | 67 | 2 2 | 2 2 | 1 1 | 0 | 0 | 0 | 0 | 0 | 0 | 0 | 0 | 0 | 0 | 0 | 0 | 0 | 0 |
| Headaches | 67 | 3 3 | 0 | 0 | 0 | 0 | 0 | 0 | 0 | 0 | 0 | 0 | 0 | 0 | 0 | 0 | 0 | 0 |
| Seizures | 0 | 0 | 0 | 0 | 0 | 0 | 0 | 0 | 0 | 0 | 0 | 0 | 0 | 0 | 0 | 0 | 0 | 0 |
| Drowsiness | 33 | 3 3 | 0 | 0 | 0 | 0 | 0 | 0 | 0 | 0 | 0 | 0 | 0 | 0 | 0 | 0 | 0 | 0 |
| Hair loss | 0 | 0 | 0 | 0 | 1 0 0 | 1 0 0 | 6 7 | 3 3 | 3 3 | 3 3 | 3 3 | 3 3 | 3 3 | 0 | 0 | 0 | 0 | 0 |
| Itchy skin | 0 | 0 | 0 | 0 | 0 | 0 | 0 | 0 | 0 | 0 | 0 | 0 | 0 | 0 | 0 | 0 | 0 | 0 |
| Weakness of legs | 67 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 |
| Bladder control | 67 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 | 1 0 0 |

### Proteomic analysis of cerebrospinal fluid

After collection of cerebrospinal fluid samples, proteins of high abundance were first depleted using high Select^{™} Top14 Abundant Protein Depletion Resin (Thermo Fisher Scientific). Purified proteins were separated with Thermo Scientific UltiMate^{™} 3000 RSLCnano System and analyzed on an Orbitrap Exploris 480 mass spectrometer using FAIMS-PulseDIA. The optimal compensation voltage (CV) was set to -48 V and -68V with a cycle time of 1 s for FAIMS experiment. MS1 resolution was set at 60,000 with a normalized AGC target of 300%. Mass range was set to 390-1010 Th. One full scan was followed by 12 windows with a resolution of 30,000 and a normalized AGC target of 200%. For part 1 method, the mass range was set to 520-630 Th at -68V and 630-750 Th at -48V. For part 2 method, the mass range was set to 400-520 Th at -68V and 750-1000 Th at -48V. Normalized HCD collision energy was 32%. The background was a human FASTA file downloaded from the UniProt proteome dataset on 26th January 2020. The database searching was performed using DIA-NN (version 1.7.8). Raw files have been deposited to the iProX: IPX0005197001.

### Statistical analysis

One-way analysis of variance (ANOVA) was used to determine the statistical differences in protein levels at different time points, and the p-values were adjusted by the Benjamini & Hochberg method. Differentially expressed proteins (ANOVA, p<0.05 after adjustment) through the therapy course were grouped into 20 clusters based on expression profiles using Mfuzz package.

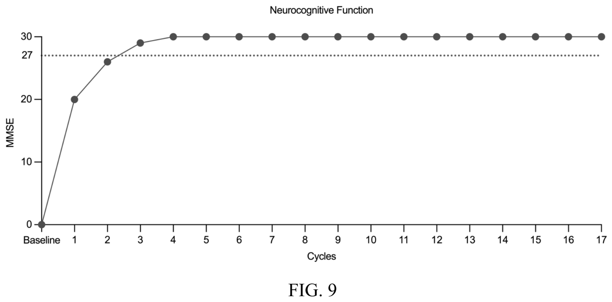

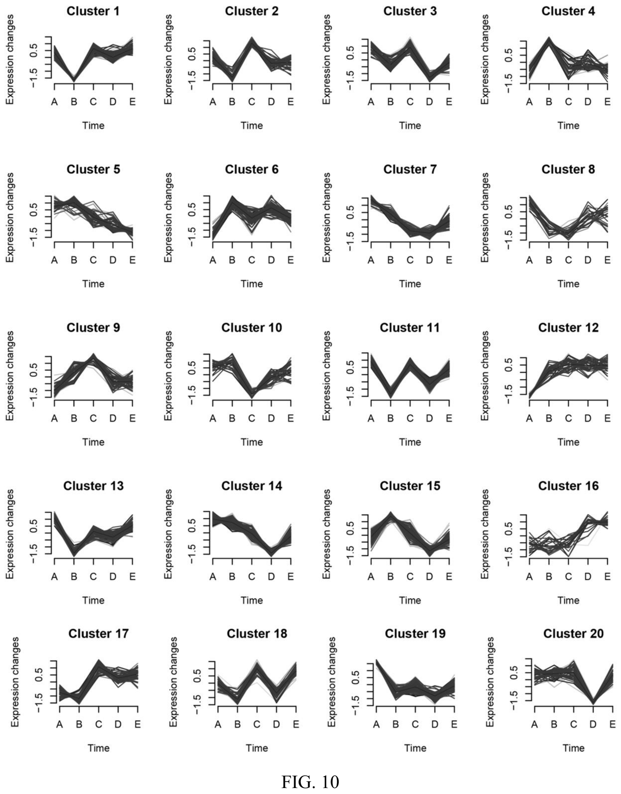

In cycle 1 of the treatment, the patient's consciousness recovered to drowsiness after surgical resection of recurrent lesion of primary thoracic tumor. However, after 10 days, the patient developed diabetes insipidus and hyponatremia due to increased volume of the metastasis lesion in the saddle area from a baseline of 1905 to 2606 mm³ (see FIG. 2a and Table 5 for details). Leptomeningeal and periventricular metastasis lesions were also progressed. After 2 cycles of treatment, the patient's electrolyte and fluid imbalance were completely resolved. His consciousness fully recovered with comprehensive improvement of neurocognitive function since cycle 3 (see FIG. 9 and Table 4 for details). When radiotherapy was completed in cycle 6, the volume of all lesions of the patient was significantly reduced, with T1 and T2 decreasing from their baseline volumes of 1,905 mm³ and 829 mm³, to 1,615 mm³ and 265 mm³, respectively, on day 122. Interestingly, in cycle 11 (on day 227), without any neurological symptom, the periventricular enhancement lesions had a slightly increase on image due to the reduced dose of panobinostat within this cycle; but in cycle 12 (on day 256), the increased lesions had a decrease in volume (see FIG. 2a, FIGs. 5-7, and Table 5 for details). Proteins in cerebrospinal fluid through the treatment course were grouped into 20 clusters based on differential expression trends (see FIG. 10 for cluster characteristics). The heatmap shows proteins that had a consistent trend in expression in cerebrospinal fluid samples collected at various time points during treatment.

### Specific results are shown below.

FIG. 3 shows the response of patient-derived tumor cells to epigenetic therapy. FIG. 3(a) is a dose-response curve showing that the change in viability of tumor cells after 72 hours of intervention with panobinostat, tazemetostat, or both in combination. The results from two independently performed assays are expressed as mean ± standard deviation (n=6). The drug solvent 0.1% DMSO was not found to affect cell viability (data not shown). Comparisons of IC50 values between drugs were performed using the extra square and F-test, *p < 0.05; ***p < 0.001. Tumor cell viability under microscopic examination after 48 hours of intervention with 1 µM panobinostat, 50 µM tazemetostat, that approximated the respective estimated IC50, or both in combination are shown on the left of FIG. 3(a). FIG. 3(b) shows effect of panobinostat and tazemetostat on migration of tumor cells in the transwell. Tumor cells that had migrated across the transwell membrane after 48 hours of intervention with 1 µM panobinostat, 50 µM tazemetostat, or both in combination were stained and observed under the microscope, with the results shown on the left of FIG. 3(b). Area of stained cells relative to field was quantified and analyzed on Image J as average from three random views. Results are expressed as mean ± standard deviations (n=6) in the bar graph. Unpaired t-tests were performed, ***p<0.001. DMSO represents dimethyl sulfoxide represents; Pan represents panobinostat; Taz represents tazemetostat; and IC50 represents half maximal inhibitory concentration.

FIG. 4 shows molecular response of patient-derived tumor cells to epigenetic therapy. FIG. 4(a) shows expression of H3K27me3 and H3K27ac in patient-derived tumor cells after 48 hours of intervention with DMSO, 1 µM panobinostat, 50 µM tazemetostat, or both. The expression levels of H3K27me3 and H3K27ac in tumor cells relative to the control group with DMSO after drug intervention are shown in FIG. 4(b) and FIG. 4(c), respectively. T test revealed a significantly increased expression of H3K27me3, significantly decreased expression of H3K27ac in tumor cells intervened with combined panobinostat and tazemetostat (n=4; N.S., not significant; **p<0.01). The expression levels of H3 and H3K27M in tumor cells were also measured by western blot assay (a). DMSO represents dimethyl sulfoxide; Pan represents panobinostat; Taz represents tazemetostat; H3K27me3 represents trimethylated H3K27; and H3K27ac represents acetylated H3K27.

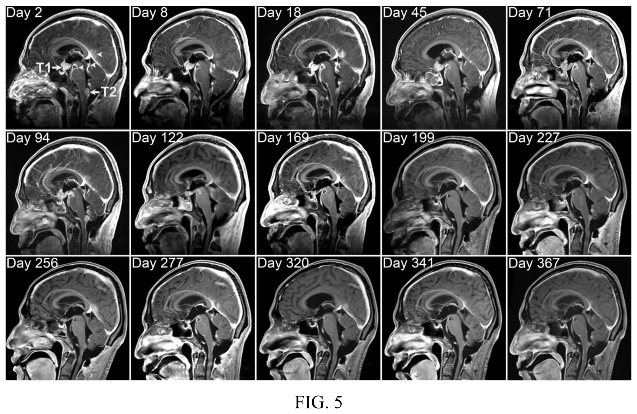

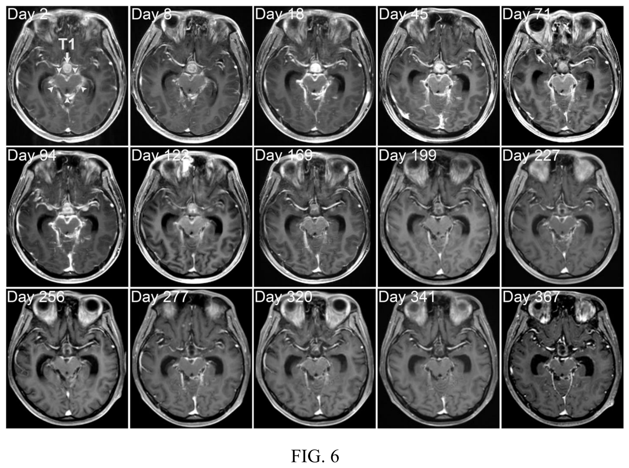

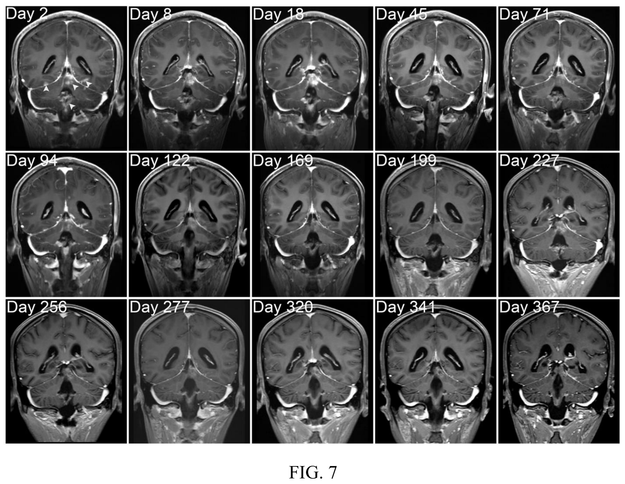

FIG. 5 shows intracranial LM on imaging (sagittal view). Sagittal view of contrast-enhanced T1-weighted MRI images are shown in the figure, which were obtained on the days indicated in the upper left of each image. Multiple linear leptomeningeal enhancement lesions are highlighted with arrowheads. T1 and T2 represent LM in the suprasaddle area and medulla oblongata tumor from baseline (on day 2). See FIG. 6 and FIG. 7 for axial and coronal images.

FIG. 6 shows intracranial LM on imaging (axial view). Axial view of contrast-enhanced T1-weighted MRI images are shown in the figure, which were obtained on the days indicated in the upper left of each image Multiple linear leptomeningeal enhancement lesions are highlighted with arrowheads. T1 represents LM in the suprasaddle area from baseline (on day 2). See FIG. 5 and FIG. 7 for sagittal and coronal images.

FIG. 7 shows intracranial LM on imaging (coronal view), with coronal view of contrast-enhanced T1-weighted MRI images are shown in the figure, which were obtained on the days indicated in the upper left of each image. Multiple linear leptomeningeal and periventricular enhancement lesions are highlighted with arrowheads from baseline MRI (on day 2). See FIG. 5 and FIG. 6 for sagittal and axial images.

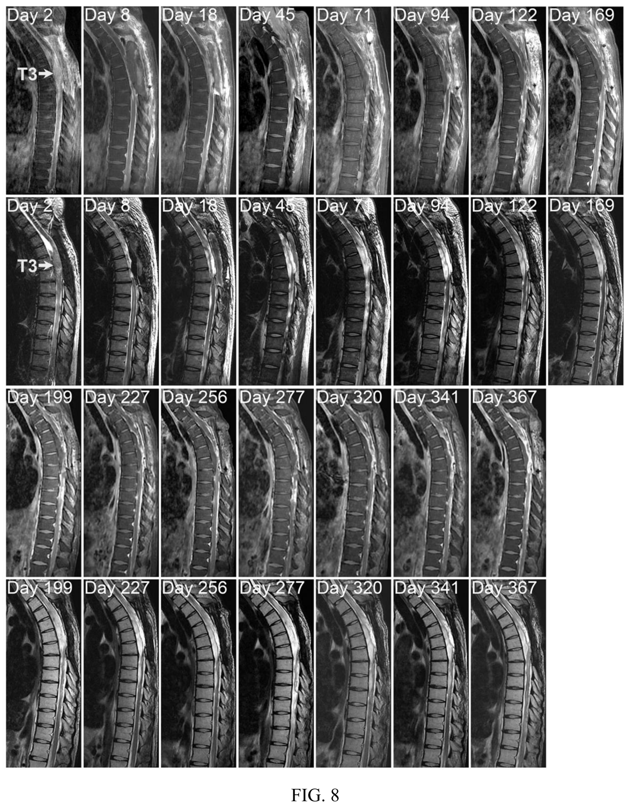

FIG. 8 shows thoracic tumor on imaging (sagittal view). Contrast-enhanced T1-weighted and T2-weighted MRI images are shown above, which were obtained on the days indicated in the upper left of each image. T3 represents recurrent thoracic tumor in baseline MRI (on day 2). See Table 5 for measurement of tumor volume on imaging.

**Table 5 Data for measurement of tumor volume on imaging (mm³)**

| Day (Cycle) | Thoracic Cord (T3) | Suprasaddle Area (T1) | Medulla Oblongata (T2) |
|---|---|---|---|
| 2 (C1) | 9057 | 1905 | 829 |
| 18 (C1) | 2538 | 2606 | 840 |
| 45 (C3) | 2842 | 2557 | 603 |
| 71 (C4) | 2476 | 2174 | 446 |
| 94 (C5) | 1896 | 2591 | 637 |
| 122 (C6) | 1394 | 1615 | 265 |
| 169 (C9) | 459 | 1078 | 77 |
| 199 (C10) | 454 | 707 | 61 |
| 227 (C11) | 446 | 864 | 66 |
| 256 (C12) | 442 | 803 | 59 |
| 277 (C13) | 435 | 508 | 43 |
| 320 (C15) | 433 | 270 | 21 |
| 341 (C16) | 346 | 177 | 18 |
| 367 (C17) | 324 | 137 | 14 |

As can be seen from Table 5, all tumor lesions in the patient showed a sustained shrinkage up to the data cut-off (on day 371), with those in the suprasaddle area (T1) and dorsal medulla (T2) measured to be 137 and 14 mm³, corresponding to >94% reduction compared with baseline volume. Enhancement signals in the leptomeningeal/ spinal meninges and periventricular were no longer detectable on enhanced MRI imaging on day 367 (FIGs 5-8). The patient had significantly improved life quality through the treatment and went back to normal life activities at the cycle 11 of treatment (FIG. 9 and Table 4).

FIG. 9 shows the results of neurocognitive function score. Neurocognitive function of the patient was assessed according to the Mini-Mental State Examination (MMSE) with scores ranging from 0 to 30. A score of ≥ 27 points indicates a normal cognition.

FIG. 10 shows the clustering trends of differential protein expression in cerebrospinal fluid. Following depletion of proteins of high abundance, proteins with differential expression in the cerebrospinal fluid through the treatment course (ANOVA adjusted p<0.05) were grouped into 20 clusters based on patterns of relative expression using Mfuzz package. Trends in relative expression of proteins in all 20 clusters are shown in FIG. 10.

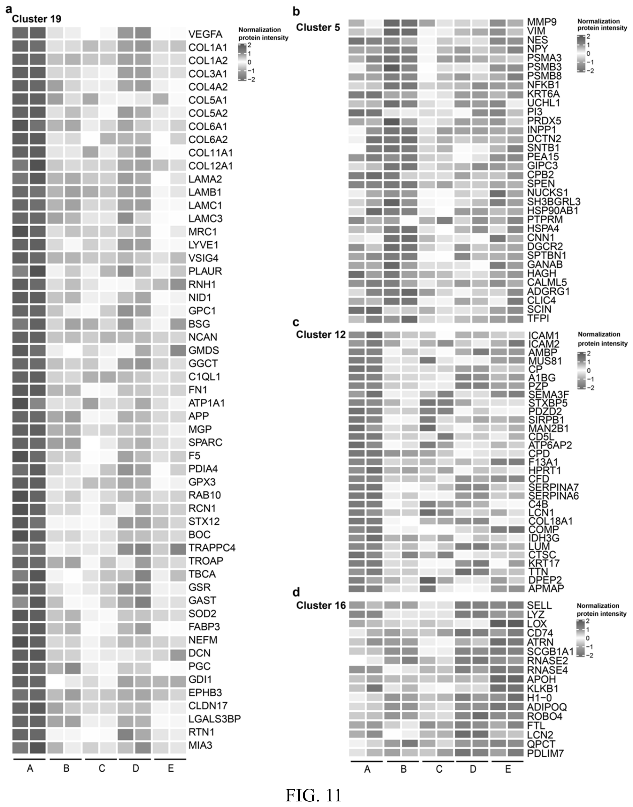

FIG. 11 shows the full list of differential protein expression in cerebrospinal fluid. FIG. 11a-d show heatmaps illustrating the relative expression of proteins in cerebrospinal fluid samples collected at various time points during treatment (see FIG. 2b for the collection time). Proteins differentially expressed through the therapy course (ANOVA adjusted p<0.05) were grouped into 20 clusters based on expression profiles using Mfuzz package (see FIG. 9 for cluster characteristics).

Samples of cerebrospinal fluid (CSF) from this patient were collected on days 26, 72, 169, 321, and 340, and subjected to proteome analysis by mass spectrometry. A total of 1812 proteins were identified, of which 1118 proteins were differentially expressed across the time points and clustered into 20 groups based on expression patterns (FIG. 10 and FIG. 11). Four clusters of consistently changed proteins were focused on. Results indicate that proteins associated with tumor angiogenesis, metastasis and proliferation, such as VEGFA, collagen family, and laminin family, were remarkably decreased since day 26. Proteins related to tumor invasion, migration, epithelial-mesenchymal transition and stemness, such as MMP9, VIM and NES, showed a steady decline since day 72. Interestingly, the expression of the protease inhibitor PZP was significantly increased since day 26, while proteasome subunits such as PSMA3, PSMB3 and PSMB8, had a significantly decreased expression since day 72, suggesting that the significant treatment efficacy was achieved by protease inhibition but not proteasome inhibitor marizomib. In addition, MRC1, a marker of M2-like tumor-associated macrophages; VISG4, a negative regulator of cytotoxic T lymphocytes activation; and PLAUR, a marker of immunosuppression in glioma, all showed a significantly decreased protein expression since day 26. ICAM1 and ICAM2, proteins associated with immune cell migration and activation, were significantly increased since day 72. The expression of proteins related to immune activation and enhanced immune response, such as SELL, LYZ and ATRN, was significantly increased since day 169, and maintained until the last sampling day.

This patient's tumor progressed malignantly at two new locations after data cut-off. The overall survival time of the patient was 20 months, and the survival time of the patient after epigenetic drug-based immunotherapy was 16 months.

### Example 2

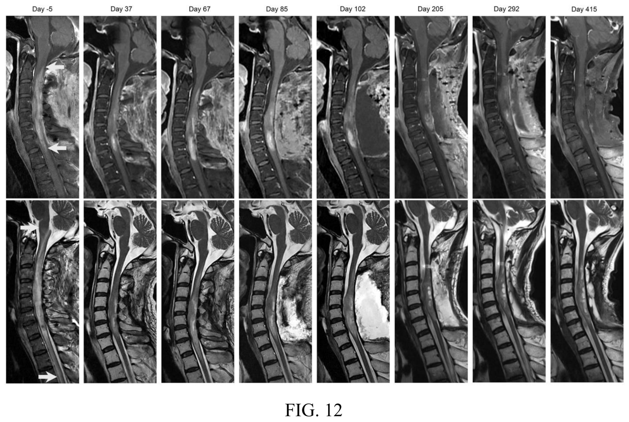

Patient 2 was a 53-year-old male with sudden paraplegia, paresthesia, and urinary incontinence as his primary clinical manifestations. Contrast-enhanced T1-weighted and T2-weighted MRI images of tumor are shown in FIG. 12. FIG. 12 shows the clinical efficacy of patient 2, in which the image acquisition date is indicated above the image. Diffuse midline glioma, H3K27 altered, in cervical cord was diagnosed from previous tumor biopsy which was performed in another hospital. The patient was admitted to Beijing Tsinghua Changgung Hospital due to radiographic and clinical signs of tumor progression (on day -5, tumor in T1-weighted images and edema in T2-weighted images were highlighted with arrows). The patient received radiotherapy (45 Gy in 25 fractions), VEGFA inhibitor bevacizumab (10 mg/kg), and concurrent immunotherapy based on the epigenetic drugs of panobinostat and tazemetostat with 3 weeks as a cycle (panobinostat 20 mg, tazemetostat 400 mg, oral on Mondays, Wednesdays, and Fridays for 2 weeks, suspension of panobinostat and tazemetostat and intravenous infusion of ipilimumab 50 mg + pembrolizumab 100 mg on week 3). Laminectomy was performed on day 71. After treatment, the patient experienced significant improvement in motor and sensory functions of his lower extremities and recovery of bladder control. These clinical improvements were accompanied by significantly decreased tumor volume in the cervical cord (on day 415). The patient survived 16+ months from diagnosis and survived 15+ months from epigenetic agent-based immunotherapy with the last follow up on October 2022.

### Example 3

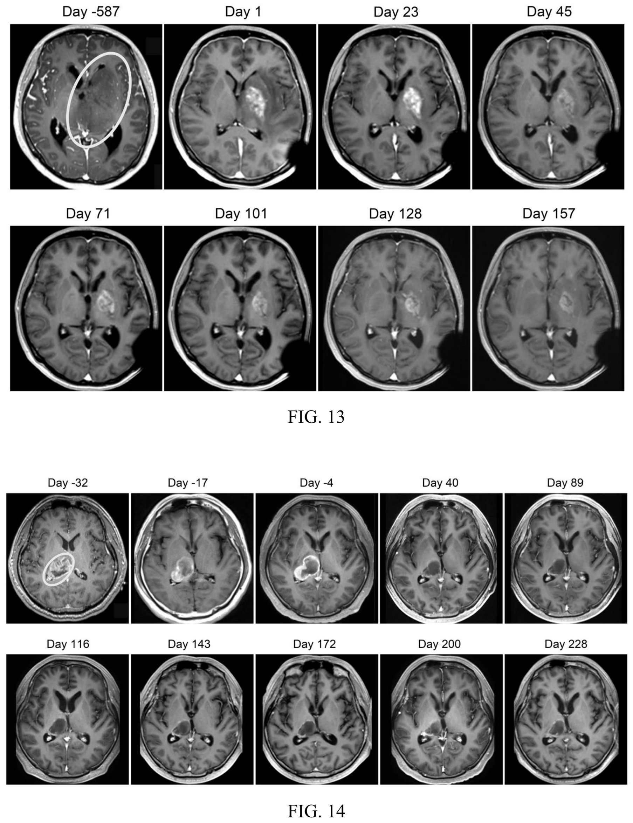

Patient 3 was a 25-year-old female with right hemiplegia and aphasia as her primary clinical manifestations. Contrast-enhanced T1-weighted MRI images of tumor are shown in FIG. 13. FIG. 13 shows the clinical course of patient 3, in which the image acquisition date is indicated above the image. Left thalamic diffuse midline glioma, H3K27 altered, was diagnosed from previous tumor biopsy which was performed in another hospital (on day -587, primary tumor was highlighted with the circle), and the patient received standard radio- and chemotherapy. Due to the progressively worsened neurological symptoms, the patient received re-radiotherapy without any clinical or radiographic improvement. MRI in Beijing Tsinghua Changgung Hospital indicated left thalamic tumor progression (on day 1). Patient 3 received immunotherapy based on epigenetic drugs of tazemetostat and panobinostat (panobinostat 20 mg, tazemetostat 400 mg, oral on Mondays, Wednesdays, and Fridays for 2 weeks, suspension of panobinostat and tazemetostat and intravenous infusion of ipilimumab 50 mg + pembrolizumab 100 mg on week 3), with concurrent VEGFA inhibitor bevacizumab (10mg/kg) with 3 or 4 weeks as a cycle. After treatment, she experienced significant improvement in right-sided motor functions and could ambulate independently. Her language skills improved in aspects including naming and fluency and she enjoyed conversations. These clinical improvements were accompanied by remarkably regressed tumor volume in the left thalamus (on day 157).

### Example 4

Patient 4 was a 44-year-old male with left upper and lower limb weakness as his primary clinical manifestations. Contrast-enhanced T1-weighted MRI images were shown in FIG. 14. FIG. 14 shows the clinical course of patient 4, in which the image acquisition date is indicated above the image. Right thalamic diffuse midline glioma, H3K27 altered, was diagnosed from previous tumor resection which was performed in another hospital (on day -32, before surgery, primary tumor was highlighted with the circle; on day -17, after surgery). He was transferred to Beijing Tsinghua Changgung Hospital due to radiographic and clinical signs of tumor progression (on day -4). He received radiotherapy (54 Gy in 27 fractions), tumor treating fields, and VEGFA inhibitor bevacizumab (10mg/kg), with concurrent immunotherapy based on epigenetic drugs of tazemetostat and panobinostat (panobinostat 20 mg, tazemetostat 400 mg, oral on Mondays, Wednesdays, and Fridays for 2 weeks, suspension of panobinostat and tazemetostat, and intravenous infusion of pembrolizumab 200 mg on week 3), with 3 or 4 weeks as a cycle. After treatment, he experienced obvious recovery of his left-sided motor functions. His quality of life improved significantly and went back to normal daily activities. He was able to climb mountains and actively engage in social activities. These clinical improvements were accompanied by remarkably diminished tumor volume in the right thalamus (on day 228).

### Example 5

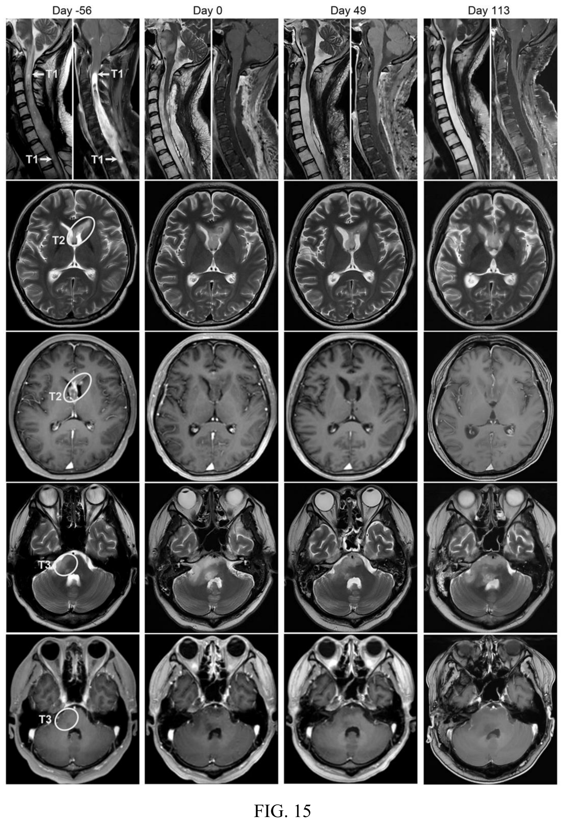

Patient 5 was a 25-year-old male with progressive weakness of left upper limb and neck pain as his primary clinical manifestations. As shown in FIG. 15, T2-weighted and contrast-enhanced T1-weighted MRI images showed the sites of 3 tumors (T1-T3). FIG. 15 shows the clinical course of patient 5, in which the image acquisition date is indicated above the image. Diffuse midline glioma, H3K27 altered, in the cervical cord was diagnosed from the first tumor resection which was performed in a hospital in Germany in September 2017. The patient received standard chemo- and radio- therapy after the first surgery. Due to tumor recurrence, the second surgery was performed in Germany in February 2019 with the same pathologic diagnosis. Without any conventional adjuvant therapy after the second surgery, the patient enrolled in a clinical trial of ONC201 in adults with recurrent H3K27M-mutant glioma from May 2019 to March 2020 in the United States. In September 2020, he presented with paraplegia, urinary and fecal incontinence, and right facial paralysis and was transferred to Beijing Tsinghua Changgung Hospital. MRI scan indicated cervical cord tumor recurrence (T1) with multifocal brain metastases (T2 and T3) (on day -56). Subtotal resection of cervical cord tumor was performed on Day -38. However, his intracranial metastases significantly progressed after the third surgery with worsened neurological symptoms (on day 0). Panobinostat and bevacizumab were given with 3 weeks as a cycle (oral administration of panobinostat 20 mg on Mondays, Wednesdays, and Fridays for 2 weeks, suspension of panobinostat and intravenous infusion of 10mg/kg bevacizumab on week 3). After 2 cycles of treatment, brain metastases of T2 and T3 remarkably regressed (on day 49) with improved neurological symptoms. Unfortunately, brain metastases eventually progressed after cycle 4. For patient 5, MRI scan on day 113 was the last imaging before his death on day 163 indicating the increased tumor burden of brain metastases. He survived 43 months from diagnosis and 5 months from treatment of panobinostat in combination with bevacizumab. As can be suggested from the response of this patient to treatment, the combination treatment of panobinostat and bevacizumab is effective in the treatment of diffuse midline glioma, H3K27 altered, but does not significantly inhibit intracranial metastasis of intramedullary tumors. Compared to the patients 1-4 above, the combination of panobinostat, tazemetostat, pembrolizumab, ipilimumab, and bevacizumab has a significantly better clinical efficacy than the combination of panobinostat and bevacizumab.

The above embodiments are only preferred embodiments of the present application. It should be noted that, for those skilled in the art, other improvements and modifications may be further made without departing from the principle of the present application, and these improvements and modifications should also be deemed as falling into the protection scope of the present application.

## Claims

1. A pharmaceutical composition comprising a HDAC inhibitor and an EZH2 inhibitor.

2. The pharmaceutical composition according to claim 1, wherein a mass ratio of the HDAC inhibitor to the EZH2 inhibitor is 100 to 200: 1000 to 4000.

3. The pharmaceutical composition according to claim 1 or 2, wherein the HDAC inhibitor is selected from the group consisting of panobinostat, vorinostat, romidepsin, belinostat, chidamide, and a combination thereof; and
the EZH2 inhibitor is tazemetostat.

4. The pharmaceutical composition according to any one of claims 1 to 3 further comprising an inhibitor selected from the group consisting of a PD-1 inhibitor, a PD-L1 inhibitor, a CTLA-4 inhibitor, a VEGF inhibitor, and a combination thereof.

5. The pharmaceutical composition according to claim 4, wherein the PD-1 inhibitor is selected from the group consisting of pembrolizumab, nivolumab, tislelizumab, toripalimab, sintilimab, camrelizumab, zimberelimab, penpulimab, serplulimab, pucotenlimab, and a combination thereof;
the PD-L1 inhibitor is selected from the group consisting of atezolizumab, durvalumab, envafolimab, sugemalimab, avelumab, and a combination thereof;
the VEGF inhibitor is selected from the group consisting of bevacizumab, ramucirumab, lucentis, aflibercept, and a combination thereof; and
the CTLA-4 inhibitor is selected from the group consisting of ipilimumab, tremelimumab, and a combination thereof.

6. The pharmaceutical composition according to claim 5, further comprising pembrolizumab, wherein a mass ratio of the HDAC inhibitor to pembrolizumab is 100 to 200: 50 to 400.

7. The pharmaceutical composition according to claim 6, further comprising ipilimumab, wherein a mass ratio of the HDAC inhibitor to ipilimumab is 100 to 200: 10 to 100.

8. The pharmaceutical composition according to claim 7, further comprising bevacizumab, wherein a mass ratio of the HDAC inhibitor to bevacizumab is 100 to 200: 300 to 1000.

9. Use of the pharmaceutical composition according to any one of claims 1 to 8 in the manufacture of a medicament against a tumor.

10. The use according to claim 9, wherein the tumor is a central nervous system tumor.

11. The use according to claim 10, wherein the tumor is a glioma.

12. The use according to claim 11, wherein the tumor is a pediatric-type diffuse high-grade glioma.

13. The use according to claim 12, wherein the tumor is a diffuse midline glioma, H3K27 altered.
